## Europäisches Patentamt

## European Patent Office

## Office européen des brevets

(19)

(11) Veröffentlichungsnummer: **0 270 999**
**B1**

(12) # EUROPÄISCHE PATENTSCHRIFT

(45) Veröffentlichungstag der Patentschrift:
**16.05.90**

(51) Int. Cl.⁵: **C07C 57/07**

(21) Anmeldenummer: **87117868.7**

(22) Anmeldetag: **03.12.87**

(54) **Verfahren zum Abtrennen von Aldehyden aus alpha, beta-olefinisch ungesättigten Carbonsäuren.**

(30) Priorität: **09.12.86 DE 3641996**

(43) Veröffentlichungstag der Anmeldung:
**15.06.88 Patentblatt 88/24**

(45) Bekanntmachung des Hinweises auf die Patenterteilung:
**16.05.90 Patentblatt 90/20**

(84) Benannte Vertragsstaaten:
**DE FR GB**

(56) Entgegenhaltungen:
**DE-A- 2 235 326**
**GB-A- 1 346 737**

(73) Patentinhaber: **BASF Aktiengesellschaft,
Carl-Bosch-Strasse 38, D-6700 Ludwigshafen(DE)**

(72) Erfinder: **Schropp, Wilhelm Karl, Dr., Im Langgewann 67,
D-6940 Weinheim(DE)**

ACTORUM AG

## Beschreibung

α,β-Olefinisch ungesättigte Carbonsäuren mit 3 bis 4 C-Atomen, wie besonders Acrylsäure und Methacrylsäure, die durch katalytische Gasphasenoxidation der entsprechenden Alkene hergestellt sind, enthalten meist von der Herstellung her geringe Mengen Carbonylverbindungen, insbesondere Aldehyde, von denen bei der weiteren Verarbeitung vor allem Furfurol stört. Weitere Carbonylverbindungen sind z.B. Acrolein, Propionaldehyd und Benzaldehyd. Beispielsweise enthält eine nach dem Verfahren der Gasphasenoxidation von Propylen hergestellte technische Acrylsäure oft 250 bis 400 ppm Furfurol. Verestert man eine derartige α,β-olefinisch ungesättigte Carbonsäure unter Zusatz von Schwefelsäure, kommt es zu störenden Ablagerungen an den Apparaturen. Außerdem kann Furfurol bei der Polymerisation der Acrylsäure regelnd wirken und so die Herstellung hochmolekularer Polymerisate erschweren.

Man hat daher schon versucht, den Gehalt an Aldehyden, insbesondere an Furfurol, durch Zusatz von Hydrazin oder wäßrigen Hydrazinlösungen und Destillation der α,β-olefinisch ungesättigten Carbonsäure drastisch zu verringern, doch gelingt es dabei nur unter Einsatz eines Überschusses von etwa 4 Mol Hydrazin unter besonderen Destillationsbedingungen eine α,β-olefinisch ungesättigte Carbonsäure mit einem Gehalt von weniger als 5ppm Furfurol herzustellen, wobei sich aber die Destillationskolonnen rasch mit Nebenprodukten belegen, so daß eine Spülung der Anlage schon nach wenigen Tagen erforderlich wird.

Außer Hydrazin hat man für die Beseitigung von Aldehyden aus roher Acrylsäure nach dem Verfahren der GB-PS 1 346 737 auch schon Phenylhydrazin, Anilin, Monoethanolamin und Ethylendiamin eingesetzt, wobei jedoch mit Hydrazin und Phenylhydrazin die kleinsten Restgehalte an Aldehyden erzielt wurden.

Es wurde nun gefunden, daß man Aldehyde aus 3 bis 4 C-Atome enthaltenden α,β-olefinisch ungesättigten Carbonsäuren durch Zusetzen von Hydrazin-Derivaten und Destillieren der ungesättigten Monocarbonsäuren mit Vorteil abtrennen kann, wenn man als Hydrazin-Derivate Aminoguanidin und/oder Aminoguanidin-Salze in Mengen von 1 bis 3 Mol/Mol Aldehyd einsetzt. Aminoguanidin und seine Salze, wie besonders das Aminoguanidin-Hydrogencarbonat sind im Handel leicht erhältlich. Sie sind weit weniger toxisch als Hydrazin und können mit einem Reinheitsgrad von etwa 99 % eingesetzt werden. Es wird daher nicht wie bei Verwendung von Hydrazin zusätzlich Wasser eingeschleppt. Bei dem neuen Verfahren wird das Aminoguanidin bzw. die Aminoguanidinsalze, wie das Hydrogencarbonat, Nitrat, Sulfat oder Chlorid, von denen das Aminoguanidin-Hydrogencarbonat vorgezogen wird in einer Menge von 1 bis 3 Mol/Mol Aldehyd eingesetzt. Meist reichen Mengen von 1,1 bis 2 Mol/Mol Aldehyd aus. Eine Reaktion des Aminoguanidins oder seiner Salze mit Acrylsäure läuft wesentlich langsamer ab, als eine entsprechende Reaktion des Hydrazins mit Acrylsäure, und die Produkte, die Aminoguanidin oder seine Salze mit den als Verunreinigungen vorliegenden Aldehyden bilden, neigen nicht zu einer Rückspaltung oder Weiterreaktion bei der destillativen Abtrennung der reinen α,β-olefinisch ungesättigten Monocarbonsäure. Schließlich bindet Aminoguanidin und dessen Salze alle als Verunreinigungen vorliegenden Aldehyde sicher und eine störende Benzaldehydbildung als Folgereaktion wird nicht beobachtet.

Bei der praktischen Durchführung des neuen Verfahrens wird das Aminoguanidin und/oder die Aminoguanidinsalze der α,β-olefinisch ungesättigten Monocarbonsäure meist in einer Menge von 1 bis 2 Mol/Mol Aldehyd, vorzugsweise in einer Menge von 1,1 bis 1,5 Mol/Mol Aldehyd zugesetzt. Zur weiteren Reinigung wird dann die α,β-olefinisch ungesättigte Monocarbonsäure destilliert, wobei im allgemeinen ein verminderter Druck von 10 bis 100 mbar angewandt wird. Die Destillation kann schon kurze Zeit nach Zugabe des Aminoguanidins bzw. von dessen Salze oder auch Stunden bzw. Tage später durchgeführt werden und bei der Destillation können zusätzlich Polymerisationsinhibitoren, wie Hydroquinon oder Hydroquinonmonomethylether in den üblichen Mengen zugesetzt werden.

Beispiel 1

Zu einer technischen Acrylsäure, die durch katalytische Gasphasenoxidation in üblicher Weise aus Propylen hergestellt ist, und die 380 ppm Furfurol (F) und 30 ppm Benzaldehyd (B) enthält, gibt man bei Raumtemperatur 1,5 bzw. 2,5 Mol/Mol Aldehyd Aminoguanidin-Hydrogencarbonat (AGHC). Die Gemische werden in zeitlichen Abständen gaschromatographisch auf ihren Aldehydgehalt untersucht und dabei folgendes Ergebnis erhalten:

Tabelle 1

| Dauer | 1,5 Mol ppm F | AGHC ppm B | 2,5 Mol ppm F | AGHC ppm B |
|---|---|---|---|---|
| 0 | 380 | 30 | 380 | 30 |
| 0,5 Stunden | 15 | 3 | < 1 | < 1 |
| 1,5 Stunden | < 1 | < 1 | < 1 | < 1 |
| 4 Stunden | < 1 | < 1 | < 1 | < 1 |
| 1 Tag | < 1 | < 1 | < 1 | < 1 |
| 5 Tage | < 1 | < 1 | < 1 | < 1 |

Destilliert man die Gemische nach 4 Stunden bei einem vermindertem Druck von 50 mbar, so erhält man ebenso wie auch nach 5 Tagen eine Acrylsäure als Destillationsprodukt, die weniger als 1 ppm Aldehyde enthält. Dabei treten keine Störungen des Destillationsprozesses auf.

Vergleichsversuch 1

Technische Acrylsäure der in Beispiel 1 angegebenen Art wird mit 1,5 bzw. 2,5 Mol Hydrazin/Mol Aldehyd versetzt, wobei das Hydrazin als 25%ige wäßrige Hydrazinhydratlösung eingesetzt wurde. Bei der gaschromatographischen Untersuchung ergaben sich folgende Aldehydreste:

Tabelle 2

| Dauer | 1,5 Mol ppm F | Hydrazin ppm B | 2,5 Mol ppm F | Hydrazin ppm B |
|---|---|---|---|---|
| 0 | 380 | 30 | 380 | 30 |
| 0,5 Stunden | 1 | 1 | 1 | 1 |
| 2 Stunden | 2 | < 1 | < 1 | < 1 |
| 5 Stunden | 10 | 1 | 3 | < 1 |
| 1 Tag | 52 | 19 | 1 | 62 |
| 5 Tage | 40 | 111 | < 1 | 178 |

Nur unmittelbar nach Zugabe von Hydrazin und sofortige Destillation des Gemisches unter vermindertem Druck (50 mbar) konnte eine praktisch aldehydfreie Acrylsäure erhalten werden, wobei jedoch schon nach kurzer Zeit eine Belegung der Destillationsapparatur mit polymeren, in Acrylsäure unlöslichen Produkten auftrat, die auch durch erhöhte Dosierung der bei der Destillation gebräuchlichen Stabilisatoren, wie Phenothiazin, nicht vermieden werden könnten.

Destilliert man das Gemisch der Acrylsäure mit dem Hydrazinhydrat später als 2 Stunden nach Zugabe des Hydrazins, so werden bei der Destillation Destillationsprodukte erhalten, die wieder Aldehyde aufweisen, wobei die Menge des Benzaldehyds, die Ausgangsmenge übersteigen kann.

Beispiel 2

Zu einer durch Gasphasenoxidation aus Methacrolein (M) hergestellten Methacrylsäure, die 20 ppm Methacrolein, 20 ppm Furfurol und 15 ppm Benzaldehyd enthält gibt man 2,5 Mol Aminoguanidin-Hydrogencarbonat/Mol Aldehyd. In dem Gemisch können kurz nach Zugabe des AGHC gaschromatographisch keine Aldehyde mehr festgestellt werden (Erfassungsgrenze 1ppm). Destilliert man das Gemisch nach 3 Stunden oder nach 3 Tagen unter vermindertem Druck (50 mbar), so erhält man eine Methacrylsäure, in der keine Aldehyde mehr gaschromatographisch nachgewiesen werden können.

Vergleichsversuch 2

Zu einer Methacrylsäure der in Beispiel 2 angegebenen Zusammensetzung gibt man 2,5 Mol Hydrazin/Mol Aldehyd in Form einer 25%igen wäßrigen Hydrazinhydrat-Lösung. Das Gemisch wird gaschromatographisch auf seinen Aldehydgehalt untersucht. Dabei werden folgende Ergebnisse erhalten:

Tabelle 3

| Dauer | ppm M | ppm F | ppm B |
|---|---|---|---|
| 0 | 20 | 20 | 15 |
| 1,5 Stunden | 6 | 4 | 2 |
| 3 Stunden | < 1 | 2 | < 1 |
| 4 Stunden | < 1 | 1 | < 1 |
| 1 Tag | < 1 | 3 | 5 |
| 5 Tage | < 1 | 7 | 11 |

Destilliert man das Gemisch aus der Methacrylsäure und der Hydrazinhydrat-Lösung, so wird nur dann ein praktisch aldehydfreie Methacrylsäure erhalten, wenn die unter vermindertem Druck durchgeführte Destillation etwa 4 Stunden nach Vermischen der Komponenten durchgeführt wird. Läßt man das Gemisch jedoch längere Zeit stehen, so steigen bei der Destillation unter vermindertem Druck die Furfurol- und Benzaldehydgehalte in den Methacrylsäure-Destillat wieder an.

**Patentansprüche**

1. Verfahren zum Abtrennen von Aldehyden aus 3 bis 4 C-Atome enthaltenden α,β-olefinisch ungesättigten Monocarbonsäuren durch Zusetzen von Hydrazin-Derivaten und Destillieren der ungesättigten Monocarbonsäuren, dadurch gekennzeichnet, daß man als Hydrazin-Derivate Aminoguanidin und/oder Aminoguanidin-Salze in Mengen von 1 bis 3 Mol/Mol Aldehyd einsetzt.

2. Verfahren nach Anspruch 1, dadurch gekennzeichnet, daß man als Aminoguanidin-Salz Aminoguanidin-hydrogencarbonat einsetzt.

**Claims**

1. A process for removing aldehydes from an α,β-olefinically unsaturated monocarboxylic acid of 3 or 4 carbon atoms by adding hydrazine derivatives and distilling the unsaturated monocarboxylic acid, wherein the hydrazine derivatives used are aminoguanidine and/or aminoguanidine salts in amounts of from 1 to 3 moles per mole of aldehyde.

2. A process as claimed in claim 1, wherein the aminoguanidine salt used is aminoguanidine hydrogencarbonate.

**Revendications**

1. Procédé pour séparer des aldéhydes d'acides monocarboxyliques à insaturation alpha, bêta-oléfinique contenant 3 à 4 atomes de carbone par addition de dérivés de l'hydrazine et distillation des acides monocarboxyliques insaturés, caractérisé en ce que l'on utilise en tant que dérivés de l'hydrazine l'aminoguanidine et/ou des sels de l'aminoguanidine en quantités de 1 à 3 moles par mole d'aldéhyde.

2. Procédé selon la revendication 1, caractérisé en ce que l'on utilise en tant que sel d'aminoguanidine le bicarbonate d'aminoguanidine.